# EUROPEAN PATENT APPLICATION

(11) **EP 2 597 101 A1**
(43) Date of publication of application: **29.05.2013**
(21) Application number: 11190133.6
(22) Date of filing: 22.11.2011
(51) Int. Cl.: C07J 75/00

(54) **Process for the preparation of drospirenone**

(71) Applicant: Industriale Chimica S.R.L., 20145 Milano (IT)
(72) Inventor: Lenna, Roberto, 20010 S. Giorgio su Legnano (IT); Vanossi, Andrea, 22046 Merone (IT)
(74) Representative: Palladino, Saverio Massimo

(57) **Abstract**

It is described a process for the preparation of Drospirenone, a synthetic steroid with progestogenic anti-mineralocorticoid and antiandrogenic activity having the formula 1 shown below, useful in the preparation of contraceptive pharmaceutical compositions, starting from 17α-(3-hydroxypropyl)-6β,7β;15β,16β-dimethylene-5β-androstan-3β,5,17β-triol.

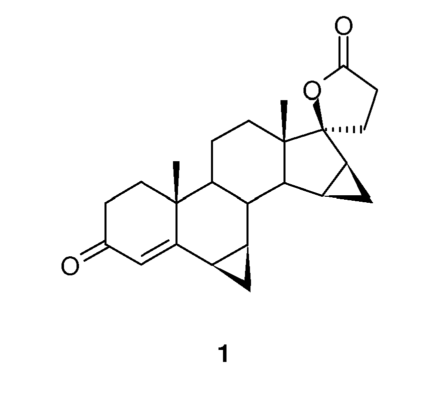

## Description

### Field of the invention

The present invention is about a process for the synthesis of steroids, and in particular a process for the industry-scale production of Drospirenone.

### Background art

The compound of formula 1 given below, having chemical name 6β,7β;15β,16β-dimethylene-3-oxo-17α-pregn-4-en-21,17-carbolactone, is commonly referred to as Drospirenone, also abbreviated DRSP (abbreviation used in the remainder of this text):

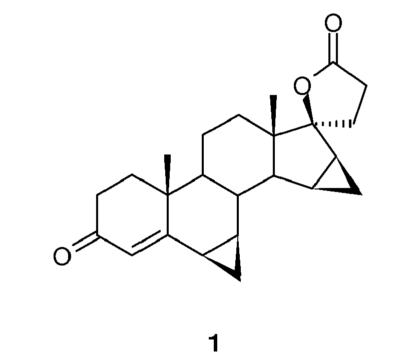

This compound is a synthetic steroid with progestogenic anti-mineralocorticoid and antiandrogenic activity; thanks to these properties, DRSP is used in the preparation of contraceptive pharmaceutical compositions for oral administration.

Various processes have been described in the literature for the preparation of DRSP.

European Patent EP no. 75189 B1 discloses a process starting with 3β,7α,15α-trihydroxy-5-androsten-17-one that, through several process steps, produces a first intermediate compound, 17α-(3-hydroxypropyl)-6β,7β;15β,16β-dimethylene-5β-androstan-3β,5,17β-triol (also referred to simply as "triol" in the following description and examples), from which the target compound is obtained via oxidation under heating with the mixture pyridine/water/chromic anhydride (Collins reactive). Actually, this last reaction passage takes place via two subsequent reactions, according to the following scheme:

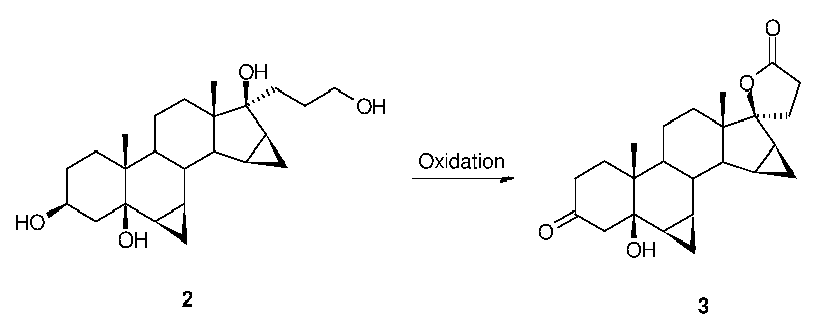

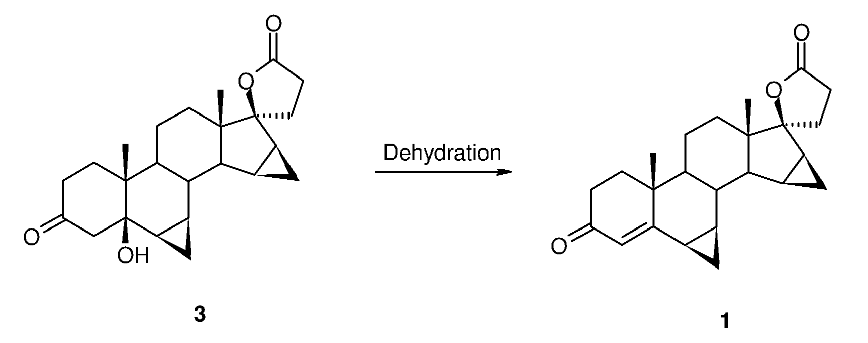

The first reaction is the oxidation of the triol (compound **2** in the scheme above) to produce 6β,7β;15β,16β-dimethylene-5β-hydroxy-3-oxo-17α-androstane-21,17-carbolactone (compound **3** in the scheme above); the latter compound is also referred to in the literature as 5β-OH-DRSP, abbreviation that will be used in the remainder of the description.

The second reaction is the dehydration of 5β-OH-DRSP, a compound presenting a beta-hydroxy-ketone structure between the positions 3 and 5 of the steroidal skeleton. It is well known that the dehydration of beta-hydroxy-ketones to yield the corresponding α-β unsaturated ketones takes place easily in the presence of acids, thanks to the higher thermodynamical stability of the latter compounds; as a consequence, the dehydration of compound (**3**) reaction above is essentially impossible to avoid in the conditions of the oxidation reaction of Patent EP no. 75189 B1, due to the presence of the Collins reactive, and takes place essentially as soon as 5β-OH-DRSP is formed, so that Drospirenone is obtained directly from the process of said patent.

The main drawback of the process described in this patent is the use of chromic anhydride that, as any Cr(VI) compound, is recognized to be carcinogenic; its use is thus subjected to extremely strict regulations, so that the measures required for use and disposal of Cr(VI) compounds rule out in practice their use in the industry.

European Patent EP 918791 B1 provides a similar process, in which the oxidation step is carried out with an oxidant (such as an organic hydroperoxides or NaBrO₃) in the presence of a ruthenium salt as a catalyst. The weaker oxidizing and acidic conditions of this synthesis route allow the isolation of the intermediate 5β-OH-DRSP which, according to the teachings of this patent, can be further reacted to give DRSP by addition of an acid. This process too has some drawbacks. First, ruthenium has toxic properties that require the adoption of measures similar to those necessary for Cr(VI), and in particular the treatment in chromatographic columns of huge volumes of reaction mixtures (the present production of DRSP is in the range of tons per year, entailing the yearly use and disposal of tons of silica and of solvents), which represents an extremely high cost in an industrial scale production. Besides, the purity degree of the raw DRSP obtained in this process is about 93%, far from an acceptable purity for applications in the pharmaceutical field, again imposing the need of long purification processes.

European patent EP 1828222 B1, in the name of the present Applicant, overcomes the problems of the processes described in the patents cited above. According to this patent, the oxidation of the triol is carried out using an oxidant, such as a hypohalide of an alkali or alkaline-earth metal, in the presence, as a catalyst, of 2,2,6,6-tetramethylpiperidine-1-oxyl radical or a derivative thereof; in this process too, the dehydration step is realized by addition of an acid directly to the reaction mixture coming from the oxidation step. This process avoids the use of Cr(VI) compounds or ruthenium salts, but still requires the use of an acid in the last step.

It is known from the literature that DRSP is instable in the presence of acids, as described e.g. in the paper "Acid catalyzed rearrangements of 15β,16β-methylene-17-alpha-pregnene-21,17-carbolactone derivatives", K. Nickisch et al., Tetrahedron Letters, 1986, vol. 27, No. 45, pp. 5463-5466. The lactone ring between the positions 17 and 21 and the three-carbon ring between the positions 6 and 7 of the molecule react in the presence of acids, forming by-products that require dedicated process steps to be eliminated, and that reduce the overall yield of the process.

It is thus an object of the present invention to provide an improved process for the preparation of Drospirenone; in particular, object of the present invention is to provide a process that avoids the use of toxic or carcinogenic metals in the oxidation reaction as well as avoiding the use of acids in the dehydration reaction that forms DRSP, thus allowing an increase in the yield and the purity of final product.

### Summary of the invention

These objects are achieved according to the present invention, with a process comprising the following steps:
a) oxidation of the compound 17α-(3-hydroxypropyl)-6β,7β;15β,16β-dimethylene-5β-androstane-3β,5,17β-triol (**2**) with a suitable oxidizing agent in the presence of a catalytic amount of 2,2,6,6-tetramethylpiperidine-1-oxyl radical or a derivative thereof;
b) removal of the solvents from the reaction mixture by distillation, obtaining an oily product;
c) addition to said oily product of a mixture of water and an organic base and heating of the resulting mixture at a temperature comprised between 35 and 90 °C, to form Drospirenone (**1**).

### Detailed description of the invention

The starting compound of the process of the invention is 17α-(3-hydroxypropyl)-6β,7β;15β,16β-dimethylene-5β-androstane-3β,5,17β-triol (**2**). This compound can be prepared according to any known method; preferably, said compound is prepared following the procedure described in patent EP 1828222 B1 or following a similar procedure.

The starting compound is then oxidized according to the procedure described in patent EP 1828222 B1, with a suitable oxidizing agent in the presence of a catalytic amount of 2,2,6,6-tetramethylpiperidine-1-oxyl radical or a derivative thereof. The oxidation of said triol could also take place according to the procedure described in Italian patent application no. M12011A001383, filed on 25 July 2011, the contents of which where rendered public in July 2011 by means of the publication of an extended abstract of the patent at the following internet link:
http://www.chemogroup.com/EN/news/Drospirenone979.pdf

The oxidation procedure of patent EP 1828222 B1 is however preferred, and the following description makes reference to the mathod of said patent.

By "suitable oxidizing agent" it is meant a compound chosen among hypohalides of alkali and alkaline-earth metals, iodine, oxygen in the presence of CuCl, potassium peroxymonosulfate KHSO₅ known commercially as Oxone^{®}, and 1,3,5-trichloro-2,4,6-triazinetrione; the preferred oxidizing agents are calcium and sodium hypochlorite. The oxidizing agent is employed in an amount, measured in equivalents, that is at least equal to the number of moles of the triol (**2**) to be oxidized, and preferably at least 1.1 times the number of moles of said triol.

The catalyst employed is 2,2,6,6-tetramethylpiperidine-1-oxyl radical, commonly referred to as "TEMPO", or derivatives thereof, such as the the 4-hydroxy-2,2,6,6-tetramethylpiperidine-1-oxyl radical, the 4-methoxy-2,2,6,6-tetramethylpiperidine-1-oxyl radical, the 4-(benzoyloxy)-2,2,6,6-tetramethylpiperidine-1-oxyl radical, the 4-(acetammido)-2,2,6,6-tetramethylpiperidine-1-oxyl radical, the 4-(amino)-2,2,6,6-tetramethylpiperidine-1-oxyl radical, the 4-(carboxy)-2,2,6,6-tetramethylpiperidine-1-oxyl radical, the 4-(cyano)-2,2,6,6-tetramethylpiperidine-1-oxyl radical, and the 4-oxo-2,2,6,6-tetramethylpiperidine-1-oxyl radical.

The catalyst is preferably employed in a molar amount comprised between about 5% and 15% the moles of the triol to be oxidized; the inventors have observed that at molar amounts below 5% the reaction takes place only with limited yield, while molar amounts in excess of 15% do not increase further the reaction yield and would thus represent a waste of catalyst.

The reaction is carried out in an organic solvent chosen among ethers such as acetone, methyl t-butyl ether and tetrahydrofuran, esters such as ethyl acetate, hydrocarbons such as toluene, halogenated hydrocarbons such as methylene chloride, and mixtures thereof, at a temperature between 0 and 40 °C, preferably between 10 and 30 °C.

Preferred reaction conditions for the oxidation reaction are the use of calcium hypochlorite in a mixture methylene chloride/tetrahydrofuran (preferably in a at least 10/1 vol/vol ratio) as the solvent, at a temperature between 20 and 30 °C in the presence of between about 10% and 14% molar (based on the triol) of TEMPO radical and in the presence of an aqueous solution of sodium bicarbonate.

The reaction mixture obtained at the end of the oxidation reaction is subjected to usual treatments for the recovery of an organic compound from an organic mixture, such as washing with water-based solutions and filtrations, and finally to distillation to remove the solvents.

After distillation, an oily product is obtained that is not further purified, and is added of a mixture water/organic base. The base that can be used may be pyridine, triethylamine, a collidine (any of the possible trimethylpiridine compounds), 1,8-diazabicyclo[5.4.0]undec-7-ene and 1,5-diazabicyclo[4.3.0]non-5-ene and derivatives thereof. The preferred base is pyridine. Pyridine is a reactive/solvent commonly used in the industry and having a low cost, stable in time and posing no particular problems in handling, and the water/pyridine mixture may be recovered at the end of the reaction by simple distillation and re-used in subsequent production runs.

The volume ratio between water and the base may vary between about 5:1 and 1:4. In case of pyridine, for instance, an azeotropic mixture, containing a volume ratio water/pyridine equal to 0.74 can be used. The inventors have noted that the water used need not be purified or distilled.

The reaction temperature may vary generally between 35 and 90 °C, depending on the base employed and its volume ratio to water: the mixtures water/base with the above mentioned bases have boiling temperatures varying with the actual composition, and the maximum temperature of the reaction must be lower than the boiling temperature of the specific water/base mixture employed.

The reaction is completed in a period comprised between 1 and 20 hours, and need not be carried out under inert atmosphere.

The raw Drospirenone product obtained with the described process has a HPLC purity degree better than 98.5%; further purification of the product, to obtain it in pharmaceutical grade, can be carried out according to usual techniques, known to those skilled in the field, such as re-crystallization, chromatography.

Thanks to the use of a blend of reactants posing no hazard, safety and flammability problems, the reaction may be carried out in a simple plant, without special safety constraints, and not requiring special heating or cooling devices. At the end of the reaction no wastes containing heavy metals, or other hazardous wastes, are produced.

The invention will be further illustrated by the following examples.

The HPLC instrument used in the examples is Agilent model 1200, and the analytical method is the one described in Ph Eur.

### EXAMPLE 1

28 g of 17α-(3-hydroxypropyl)-6β,7β;15β,16β-dimethylene-5β-androstane-3β,5,17β-triol are dissolved at 35 °C in 56 ml of tetrahydrofuran (THF) in a reaction flask. 712 ml of dichloromethane and 420 ml of an aqueous solution of NaHCO₃ (10% by weight, b.w.) are then added, obtaining a biphasic mixture that is stirred and cooled down to 10 °C.

Under stirring, TEMPO catalyst and calcium hypochlorite as oxidizing agent are added in portions, allowing the mixture to react for 1 hour after any addition and checking advancement degree of the reaction at the end of any period by thin layer chromatography (TLC). In particular, in the first dosing are added 700 mg of TEMPO and, after a few minutes, 11.2 g of calcium hypochlorite. The mixture is stirred heating it to 28-30 °C. The TLC check shows a still partial reaction. Keeping the reaction mixture under stirring and at a temperature of 28-30 °C, a second dosing of 500 mg of TEMPO and, after a few minutes 11.2 g of calcium hypochlorite, is added; the reaction is left to proceed, and after one hour a not complete reaction is observed again by TLC. Always under stirring and at a temperature of 28-30 °C, a third dosing of 140 mg of TEMPO and, after a few minutes, 4.2 g of calcium hypochlorite, is added; after one hour the TLC confirms that the reaction is complete, no starting triol being detected.

110 ml of dichloromethane is added to the reaction mixture, and the same is filtered on diatomaceous earth. The filter is rinsed with another 100 ml of dichloromethane.

The aqueous phase and the organic phase are separated, and the latter is washed, in sequence, with 520 ml of a 2% b.w. water solution of NaHSO₄ and then with 520 ml of a 4% b.w. water solution of NaCl. The pH of the final aqueous phase is 7.

The organic phase is stirred for a few minutes with 1.2 g of decoloring carbon and with 1.2 g of diatomaceous earth. The suspension is filtered, and the filter washed with 60 ml of dichloromethane.

The organic phase is distilled until a semisolid-oily residue is obtained. In the distillation flask, 280 ml of water and 280 ml of pyridine are added. Under stirring, the mixture is heated to 45-50 °C during 16 h. At the end of this period, a TLC check confirms that the reaction is complete: a unitary spot is obtained, having a Rf corresponding to DRSP (checked with a spot of pure product on the same TLC)

310 ml of water/pyridine mixture are distilled off under reduced pressure (T of the distilling vapors = 24-25 °C). 460 ml of water is added and the distillation continued, removing further 50 ml of solvent. 75 ml of isopropyl acetate is added and the mixture is stirred cooling the system with a water-ice bath, observing the formation of a solid in the distillation flask, which is filtered and dried under reduced pressure.

12,3 g of Drospirenone with HPLC purity of 98.7% are obtained. By concentrating the organic phase another 6.1 g of Drospirenone are obtained, for a total yield of recovered product of 70% referred to the starting triol.

### EXAMPLE 2

The oxidation reaction of Example 1 is repeated, using 1 g of the starting triol; reactants and solvents are used in the same proportions to the triol as in Example 1.

At the end of the oxidation, in the reaction flask 10 ml of water and 20 ml of pyridine are added, and the mixture is stirred at a T comprised between 58 and 62 °C during 7 hours. After this period, a TLC check confirms that the reaction is complete (single spot with Rf corresponding to DRSP).

The reaction mixture is distilled under reduced pressure obtaining 1.023 g of raw product which, at a HPLC titration, contain 685 mg of Drospirenone with HPLC purity of 94.7%.

The reaction yield, referred to the starting triol, is 73%.

### EXAMPLE 3

The procedure of Example 2 is repeated, adding in this case, at the end of the oxidation reaction, 10 ml of water and 15 ml of pyridine. After the TLC check, confirming that the reaction is complete (single spot with Rf corresponding to DRSP), the reaction mixture is distilled under reduced pressure obtaining 1.027 g of raw product which, at a HPLC titration, contain 684 mg of Drospirenone with HPLC purity of 92.2%.

The reaction yield, referred to the starting triol, is 73%.

### EXAMPLE 4

The procedure of Example 2 is repeated, adding in this case, at the end of the oxidation reaction, 10 ml of water and 10 ml of pyridine.

After the TLC check, confirming that the reaction is complete (single spot with Rf corresponding to DRSP), the reaction mixture is distilled under reduced pressure obtaining 1.005 g of raw product which, at a HPLC titration, contain 679 mg of Drospirenone with HPLC purity of 92.0%.

The reaction yield, referred to the starting triol, is 72%.

### EXAMPLE 5

The procedure of Example 2 is repeated, adding in this case, at the end of the oxidation reaction, 10 ml of water and 5 ml of pyridine.

After the TLC check, confirming that the reaction is complete (single spot with Rf corresponding to DRSP), the reaction mixture is distilled under reduced pressure obtaining 995 g of raw product which, at a HPLC titration, contain 652 mg of Drospirenone with HPLC purity of 92.0%.

The reaction yield, referred to the starting triol, is 69%.

### EXAMPLE 6

The procedure of Example 2 is repeated, adding in this case, at the end of the oxidation reaction, 10 ml of water and 2.5 ml of pyridine, and heating the mixture in this case for 15 hours.

After the TLC check, confirming that the reaction is complete (single spot with Rf corresponding to DRSP), the reaction mixture is distilled under reduced pressure obtaining 1.012 g of raw product which, at a HPLC titration, contain 579 mg of Drospirenone with HPLC purity of 90.0%.

The reaction yield, referred to the starting triol, is 61 %.

### EXAMPLE 7

The procedure of Example 2 is repeated, adding in this case, at the end of the oxidation reaction, 10 ml of water and 10 ml of pyridine, and heating the mixture in this case at a temperature comprised between 38 and 42 °C for 20 hours.

After the TLC check, confirming that the reaction is complete (single spot with Rf corresponding to DRSP), the reaction mixture is distilled under reduced pressure obtaining 895 g of raw product which, at a HPLC titration, contain 329 mg of Drospirenone with HPLC purity of 95.0%.

The reaction yield, referred to the starting triol, is 35%.

### EXAMPLE 8

The procedure of Example 7 is repeated, heating the mixture in this case at a temperature comprised between 78 and 82 °C for 15 hours.

After the TLC check, confirming that the reaction is complete (single spot with Rf corresponding to DRSP), the reaction mixture is distilled under reduced pressure obtaining 1.002 g of raw product which, at a HPLC titration, contain 652 mg of Drospirenone with HPLC purity of 88.0%.

The reaction yield, referred to the starting triol, is 69%.

### EXAMPLE 9 (COMPARATIVE)

The oxidation reaction of Example 2 is repeated.

At the end, to the oxidation product, only 20 ml of pyridine (no water) is added.

The mixture is heated under stirring at a temperature comprised between 48 and 52 °C for 18 hours.

At the TLC check, the reaction results to be largely incomplete: the TLC shows two main spots, the minority one corresponding to Drospirenone. The solvent is distilled, recovering 1.027 mg of residue that shows the same TLC profile as the previous control.

### EXAMPLE 10 (COMPARATIVE)

The oxidation reaction of Example 1 is repeated, using 500 mg of starting triol; reactants and solvents are used in the same proportions to the triol as in Example 1.

At the end of the oxidation, in the reaction flask 5 ml of ethyl alcohol and 50 mg of sodium methylate (CH₃ONa) are added, and the mixture is stirred at a T comprised between 38 and 42 °C during 1 hour. After this period, a TLC check confirms that the reaction is complete (single spot with Rf corresponding to DRSP). Acetic acid is added bringing the pH to a value of about 6, than the solvent is distilled under reduced pressure.

The residue is taken with 5 ml of water and 5 ml of dichloromethane. The organic phase is concentrated to dryiness under reduced pressure.

The obtained product (395 mg), checked with TLC, shows more than one spot. The DRSP content of the sample, measured by HPLC against a reference sample, results equal to 36% b.w. with a purity degree of 59%.

The reaction yield, referred to the starting triol, is 30%.

### EXAMPLE 11

The oxidation reaction of Example 1 is repeated, preparing a starting mixture with 28 g of starting triol dissolved in 60 ml of THF at 35 °C, to which 710 ml of dichloromethane and 420 ml of an aqueous solution of NaHCO₃ (10% by weight, b.w.) are added, obtaining a biphasic mixture that is stirred and cooled down to 10 °C.

The addition of calcium hypochlorite and TEMPO is carried out in three subsequent dosings as described in Example 1, under stirring and keeping the reacting mixture at a temperature between 28-30 °C, with a first dosing of 700 mg of TEMPO and, after a few minutes, 11.2 g of calcium hypochlorite (partial reaction), a second dosing of 510 mg of TEMPO and, after a few minutes, 11.4 g of calcium hypochlorite (partial reaction), and a third dosing of 130 mg of TEMPO and, after a few minutes, 4.0 g of calcium hypochlorite; after 1 h from the last dosing, the reaction is complete, no unreacted starting triol is observed by TLC.

120 ml of dichloromethane is added to the reaction mixture, and the same is filtered on diatomaceous earth. The filter is rinsed with another 100 ml of dichloromethane.

The aqueous phase and the organic phase are separated, and the latter is washed, in sequence, with 520 ml of a 2% b.w. water solution of NaHSO₄ and then with 520 ml of a 4% b.w. water solution of NaCl. The pH of the final aqueous phase is 7.

The organic phase is stirred for a few minutes with 1.2 g of decoloring carbon and with 1.5 g of diatomaceous earth. The suspension is filtered, and the filter washed with 60 ml of dichloromethane.

The organic phase is distilled briefly, until a semisolid-oily residue is obtained.

In the distillation flask, 280 ml of water and 140 ml of pyridine are added. Under stirring, the mixture is heated to 58-62 °C during 20 h. At the end of this period, a TLC check confirms that the reaction is complete: a unitary spot is obtained, having a Rf corresponding to DRSP.

200 ml of water/pyridine mixture are distilled off under reduced pressure (T of the distilling vapors = 24-25 °C).

200 ml of water is added, the mixture is cooled down to 20-25 °C, and extracted with 300 ml of dichloromethane. After water rinsing, the organic phase is filtered and then treated with 1.2 g of decoloring carbon, 1.2 g of diatomaceous earth, and 4.8 g of sodium sulfate (Na₂SO₄) and filtered again. The residual pyridine is eliminated by distillation with methyl isobutyl ketone (MIBK).

The residue is crystallized from isopropyl acetate, providing 14.5 g of Drospirenone (constant weight after drying at 50 °C under reduced pressure) which shows a HPLC purity of 98.9%.

From the aqueous mother solution, by further crystallization, another 4.36 g of DRSP are obtained.

The residue of the crystallization steps, checked by TLC, shows the presence of DRSP. After chromatography a further 1.4 g of DRSP is obtained *for a* total yield of recovered product of 78% referred to the starting triol

### EXAMPLE 12

The oxidation reaction of Example 1 is repeated, using 500 mg of the starting triol; reactants and solvents are used in the same proportions to the triol as in Example 1.

At the end of the oxidation, in the reaction flask 5 ml of water and 15 ml of pyridine are added, and the mixture is stirred at a T comprised between 48 and 52 °C during 15 hours. After this period, a TLC check confirms that the reaction is complete (single spot with Rf corresponding to DRSP).

The reaction mixture is distilled under reduced pressure obtaining 502 mg of raw product which, at the HPLC analysis, is confirmed to be Drospirenone with purity of 92.0% (λ= 254 nm, non-integrated pyridine signal)

The DRSP content of the sample, measured by HPLC against reference sample, is 66% b.w.

The reaction yield, referred to the starting triol, is 70%.

### EXAMPLE 13 (COMPARATIVE)

The oxidation reaction of Example 12 is repeated.

At the end of the oxidation, in the reaction are added flask 20 ml of water (a suspension is formed). The mixture is stirred at a T comprised between 48 and 52 °C during 16 hours. After this period, a TLC check reveals that the reaction is highly incomplete: the TLC shows two spots, the minority one, sparingly seen, corresponding to the Drospirenone (traces).

### EXAMPLE 14

10 g of 17α-(3-hydroxypropyl)-6β,7β;15β,16β-dimethylene-5β-androstane-3β,5,17β-triol are suspended at 25 °C in 240 ml of dichloromethane with 500 mg of an aqueous solution of NH₂OH (50% by weight) and 800 mg of TEMPO.

The mixture is stirred at P = 6 bar at T = 80 °C for 4 h.

The pressure of 6 bar is obtained charging pure oxygen in the reactor.

The TLC check shows a complete reaction: the starting triol is no more detectable and the TLC shows two main spots, one corresponding to drospirenone.

The reaction mixture is filtered on diatomaceous earth.

The organic phase is washed, in sequence, with 200 ml of a 2% b.w. water solution of NaHSO₄ and then with 200 ml of a 4% b.w. water solution of NaCl.

The organic phase is stirred for a few minutes with 1.0 g of decoloring carbon and 1.0 g of diatomaceous earth. The suspension is filtered, and the filter washed with 60 ml of dichloromethane.

The organic phase is distilled until a semisolid-oily residue is obtained. In the distillation flask, 100 ml of water and 100 ml of pyridine are added. Under stirring, the mixture is heated to 45-50 °C during 16 h. At the end of this period, a TLC check confirms that the reaction is complete: a unitary spot is obtained, having a Rf corresponding to DRSP (checked with a spot of pure product on the same TLC).

90 ml of water/pyridine mixture are distilled off under reduced pressure

150 ml of water is added, the mixture is then cooled to 20-25 °C, and extracted with 300 ml of dichloromethane. After water rinsing, the organic phase is filtered and then treated with 1.0 g of decoloring carbon, 1.0 g of diatomaceous earth, 2.5 g of sodium sulfate (Na₂SO₄) and filtered again. The residual pyridine is eliminated by distillation with methyl isobutyl ketone (MIBK).

After drying at reduced pressure 4.9 g of drospirenone are obtained (checked by TLC against a spot of pure drospirenone).

## Claims

1. Process for the preparation of Drospirenone comprising the following steps:
a) oxidation of the compound 17α-(3-hydroxypropyl)-6β,7β;15β,16β-dimethylene-5β-androstane-3β,5,17β-triol (**2**) with a suitable oxidizing agent in the presence of a catalytic amount of 2,2,6,6-tetramethylpiperidine-1-oxyl radical or a derivative thereof;
b) removal of the solvents from the reaction mixture by distillation, obtaining an oily product;
c) addition to said oily product of a mixture of water and an organic base and heating of the resulting mixture at a temperature comprised between 35 and 90 °C, to form Drospirenone (**1**).

2. Process according to claim 1, in which steps a), b) and c) take place in a single reaction container, with no isolation and purification of intermediate compounds.

3. Process according to any one of the preceding claims, in which the oxidizing agent is chosen among hypohalides of alkali and alkaline-earth metals, iodine, oxygen in the presence of CuCl, potassium peroxymonosulfate KHSO₅ known commercially as Oxone^{®}, and 1,3,5-trichloro-2,4,6-triazinetrione.

4. Process according to claim 3, in which said oxidizing agent is chosen between calcium and sodium hypochlorite.

5. Process according to any one of the preceding claims, in which the oxidizing agent is employed in an amount, measured in equivalents, at least equal to the number of moles of the triol (**2**) to be oxidized.

6. Process according to claim 5, in which said oxidizing agent is employed in an amount, measured in equivalents, at least 1.1 times the number of moles of the triol (**2**) to be oxidized.

7. Process according to any one of the preceding claims, in which the oxidation catalyst employed is selected among the 2,2,6,6-tetramethylpiperidine-1-oxyl radical, the 4-hydroxy-2,2,6,6-tetramethylpiperidine-1-oxyl radical, the 4-methoxy-2,2,6,6-tetramethylpiperidine-1-oxyl radical, the 4-(benzoyloxy)-2,2,6,6-tetramethylpiperidine-1-oxyl radical, the 4-(acetammido)-2,2,6,6-tetramethylpiperidine-1-oxyl radical, the 4-(amino)-2,2,6,6-tetramethylpiperidine-1-oxyl radical, the 4-(carboxy)-2,2,6,6-tetramethylpiperidine-1-oxyl radical, the 4-(cyano)-2,2,6,6-tetramethylpiperidine-1-oxyl radical, and the 4-oxo-2,2,6,6-tetramethylpiperidine-1-oxyl radical.

8. Process according to any one of the preceding claims, in which the catalyst is employed in a molar amount comprised between about 5% and 15% the moles of the triol (**2**) to be oxidized.

9. Process according to any one of the preceding claims, in which the oxidation reaction of step a) is carried out in a solvent chosen among ethers, esters, hydrocarbons, halogenated hydrocarbons and mixtures thereof, at a temperature between 0 and 40 °C.

10. Process according to claim 9, in which said oxidation step a) is carried out with calcium hypochlorite as the oxidizing agent, a mixture methylene chloride/tetrahydrofuran as the solvent, at a temperature between 20 and 40 °C in the presence of 2,2,6,6-tetramethylpiperidine-1-oxyl radical as the catalyst and in the presence of an aqueous sodium bicarbonate solution.

11. Process according to any one of the preceding claims, in which the base used in mixture with water in the dehydration step c) is selected among pyridine, triethylamine, a collidine, 1,8-diazabicyclo[5.4.0]undec-7-ene and 1,5-diazabicyclo[4.3.0]non-5-ene and derivatives thereof, and the volume ratio between water and the base is comprised between about 5:1 and 1:4.

12. Process according to claim 11, in which said base is pyridine.

13. Process according to any one of the preceding claims, in which the dehydration dehydration step c) lasts between 1 and 20 hours.
